# EUROPEAN PATENT APPLICATION

(11) **EP 4 393 374 A1**
(43) Date of publication of application: **03.07.2024**
(21) Application number: 22873038.8
(22) Date of filing: 26.09.2022
(51) Int. Cl.: A61B 5/00, A61B 5/01, A61B 5/02, A61B 5/08, A61B 5/107, A61B 5/11

(54) **INFORMATION PROCESSING SYSTEM, PROGRAM, AND INFORMATION PROCESSING METHOD**

(30) Priority: 27.09.2021 JP 2021156953
(71) Applicant: Terumo Kabushiki Kaisha, Tokyo 151-0072 (JP)
(72) Inventor: HONMA, Yasuyuki, Ashigarakami-gun, Kanagawa 259-0151 (JP)
(74) Representative: Casalonga
(86) International application number: PCT/JP2022/035749
(87) International publication number: WO 2023/048287

(57) **Abstract**

An information processing system and the like that can be easily introduced and can detect biological information in a preferred manner are provided.

The information processing system includes: a holding device including a holding unit that holds a portable information processing terminal, and a communication unit that communicates with the portable information processing terminal, the portable information processing terminal including a first detector that detects biological information; and a control unit that performs control for acquiring the biological information. In a case where the portable information processing terminal is held by the holding unit, and communication between the portable information processing terminal and the holding device has been established via the communication unit, the control unit acquires the biological information detected by the first detector.

## Description

### Technical Field

The present technology relates to an information processing system, a program, and an information processing method.

### Background Art

There have been monitor devices that monitor biological information such as respiratory conditions and pulses. For example, Patent Literature 1 discloses a biological information monitoring device that monitors biological information such as pulses by emitting high-frequency electromagnetic waves, detecting reflected waves scattered on a biological surface of a person, and calculating the temporal variation in the biological surface.

### Citation List

### Patent Literature

Patent Literature 1: JP 2005-270570 A

### Summary of Invention

### Technical Problem

The monitoring device disclosed in Patent Literature 1 has a problem in that it is difficult to easily introduce the monitoring device, because a detector needs to be provided in the monitoring device and leads to an increase in cost. Furthermore, in a case where the monitoring device is installed in a residence or the like including a plurality of rooms, biological information cannot be detected in a preferred manner in a room that is outside the electromagnetic wave radiation range.

An object of the present disclosure is to provide an information processing system and the like that can be easily introduced and detect biological information in a preferred manner.

### Solution to Problem

An information processing system according to an aspect of the present disclosure includes: a holding device including a holding unit that holds a portable information processing terminal, and a communication unit that communicates with the portable information processing terminal, the portable information processing terminal including a first detector that detects biological information; and a control unit that performs control for acquiring the biological information. In a case where the portable information processing terminal is held by the holding unit, and communication between the portable information processing terminal and the holding device has been established via the communication unit, the control unit acquires the biological information detected by the first detector.

A program according to an aspect of the present disclosure is for causing a computer to perform a process of acquiring biological information detected by a first detector, in a case where a portable information processing terminal including the first detector that detects the biological information is held by a holding device including a holding unit that holds the portable information processing terminal and a communication unit that communicates with the portable information processing terminal, and communication between the portable information processing terminal and the holding device has been established via the communication unit.

An information processing method according to an aspect of the present disclosure is implemented by a computer to perform a process of acquiring biological information detected by a first detector, in a case where a portable information processing terminal including the first detector that detects the biological information is held by a holding device including a holding unit that holds the portable information processing terminal and a communication unit that communicates with the portable information processing terminal, and communication between the portable information processing terminal and the holding device has been established via the communication unit.

### Advantageous Effects of Invention

According to the present disclosure, it is possible to provide an information processing system and the like that can be easily introduced and detect biological information in a preferred manner.

### Brief Description of Drawings

Fig. 1 is a schematic diagram of an information processing system according to a first embodiment.
Fig. 2 is a block diagram illustrating the configuration of a holding device.
Fig. 3 is a block diagram illustrating the configuration of a portable information processing terminal.
Fig. 4 is a block diagram illustrating the configuration of an information processing device.
Fig. 5A is an explanatory diagram illustrating examples of record layouts in a holding device DB, a subject DB, and a biological information DB.
Fig. 5B is an explanatory diagram illustrating examples of record layouts in a holding device DB, a subject DB, and a biological information DB.
Fig. 5C is an explanatory diagram illustrating examples of record layouts in a holding device DB, a subject DB, and a biological information DB.
Fig. 6 is a flowchart illustrating information processing procedures according to the first embodiment.
Fig. 7 is a flowchart illustrating information processing procedures according to the first embodiment.
Fig. 8 is a flowchart illustrating information processing procedures according to a second embodiment.
Fig. 9 is a flowchart illustrating information processing procedures according to a third embodiment.
Fig. 10 is a schematic diagram of an information processing system according to a fourth embodiment.
Fig. 11 is a flowchart illustrating information processing procedures according to the fourth embodiment.

### Description of Embodiments

Specific examples of information processing systems, programs, and information processing methods according to embodiments of the present invention will be described below with reference to the drawings. Note that the present invention is not limited by these examples but is disclosed in the claims, and is intended to include all changes within the meaning and scope equivalent to the claims. Further, at least some of the embodiments described below may be combined as appropriate.

Note that the sequences in the embodiments described below are not limited, and each processing procedure may be carried out in a changed order, or a plurality of processes may be performed in parallel, within a range without contradictions. The processing subject in each process is not limited, and processes to be performed by the respective devices may be performed by some other device within a range without contradictions.

### First Embodiment

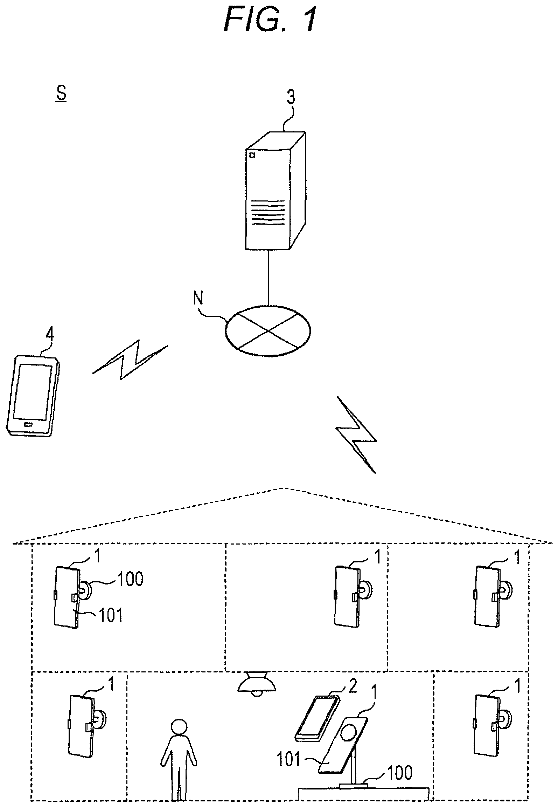

Fig. 1 is a schematic diagram of an information processing system S according to a first embodiment. The information processing system S includes a holding device 1 according to the first embodiment. The holding device 1 is wirelessly connected to a portable information processing terminal 2 and an information processing device 3, and can transmit and receive various kinds of information. The information processing device 3 is also wirelessly connected to a communication terminal 4, and can transmit and receive various kinds of information.

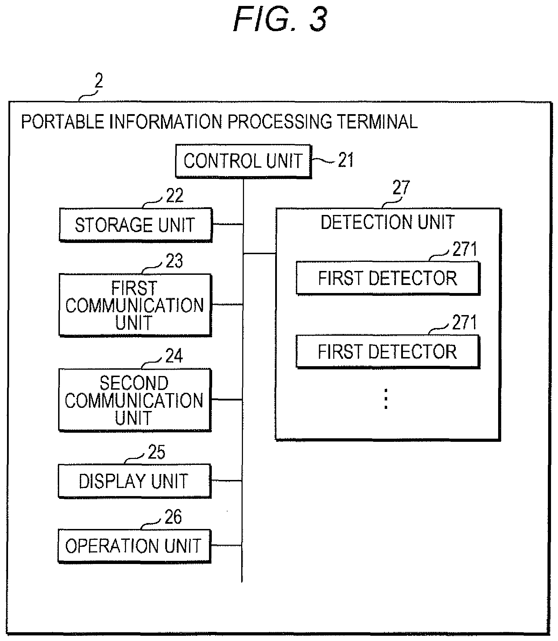

The holding device 1 functions as a biological information detecting device that acquires biological information about a subject (a biological object) via the portable information processing terminal 2. The holding device 1 is provided in a room of a residence of the subject, for example, and is used, with the portable information processing terminal 2 of the subject being set to the holding device 1. The portable information processing terminal 2 is a portable-type information processing terminal device that is used by the subject, and includes a first detector 271 (see Fig. 3) that detects a communication function and biological information. The portable information processing terminal 2 is a smartphone, a tablet terminal, or the like, for example. The information processing device 3 is a server computer, a quantum computer, or the like, for example. The information processing device 3 is a device that determines the possibility of a disease in the subject, using biological information. The communication terminal 4 is a communication terminal that is used by a person other than the subject, such as medical personnel or a family member of the subject, for example. Note that the respective devices may be configured to be connected by a connecting terminal or a cable.

The holding device 1 is preferably installed at a plurality of locations in the residence, such as an entrance, a living room, a bedroom, a toilet, a dressing room, and a bathroom of the residence of the subject, for example. When moving in the residence, the subject sets the portable information processing terminal 2 onto a holding unit 101 of the holding device 1 installed at each place as a destination of movement. The holding device 1 also functions as a temporary stand for the portable information processing terminal 2.

The holding device 1 includes a main body 100 and a holding unit 101 attached to the main body 100. The portable information processing terminal 2 is held by the holding unit 101. The holding device 1 may be secured to an indoor wall, furniture, a mirror, or the like with a fixture, for example, or may be disposed on a desk, a bedside, or the like in a non-fixed manner.

The shape of the holding unit 101 that holds the portable information processing terminal 2 is not limited to a particular shape, as long as the portable information processing terminal 2 can be held rotatably in horizontal and vertical directions. The holding unit 101 includes a support extending from the main body 100, and a rectangular holding panel continuous with the support, for example. A long side of the holding panel may be provided with protruding portions protruding at a substantially right angle, and the portable information processing terminal 2 may be held between the protruding portions. The holding unit 101 may include a fitting portion into which part of the portable information processing terminal 2 can be fitted, and hold the portable information processing terminal 2 as the portable information processing terminal 2 is fitted into the fitting portion. The holding unit 101 may include an attracting unit containing a magnet, for example, and hold the portable information processing terminal 2 by attraction caused by the magnetic force between the attracting unit and a magnet contained in the portable information processing terminal 2.

In a case where the portable information processing terminal 2 is set onto the holding device 1, the holding device 1 turns on a biological information detection mode of the portable information processing terminal 2, and acquires, via the portable information processing terminal 2, detected biological information (detection values) from the first detector 271 of the portable information processing terminal 2. The holding device 1 transmits the acquired biological information to the information processing device 3. The information processing device 3 determines the possibility of a disease on the basis of the biological information, and outputs a result of the determination to the holding device 1, the portable information processing terminal 2, or the communication terminal 4.

The first detector 271 of the portable information processing terminal 2 may include a camera, a microphone, an infrared sensor (a laser sensor), a millimeter-wave sensor, or the like, for example. The portable information processing terminal 2 may include a first detector (a sensor device) 271 that is one of the sensors mentioned above, or may include a plurality of first detectors 271.

The biological information in the present embodiment may include at least one piece of information detected by the first detector 271 described above, such as an image of the face, a hand, or a foot of the subject, pulsation of the heart or blood vessels (a heartbeat or pulse), respiration, body temperature, voice, and the like.

The possibility of a disease in the present embodiment includes the possibility of a disease related to an abnormality of the heart or blood vessels. An abnormality of the heart in the present embodiment refers to an abnormality observed in the heart. Abnormalities of blood vessels include abnormalities observed in blood vessels, and abnormalities caused in internal organs, organs, and body parts due to an abnormality in blood flow. Examples of such diseases related to an abnormality of the heart or blood vessels include cerebral infarction, cerebral hemorrhage, arteriosclerosis, heart failure, foot infarction (for example, critical limb ischemia), varicose vein, and deep vein thrombus. The information processing device 3 integrates biological information transmitted from a plurality of holding devices 1, which is biological information detected at a plurality of detection points, and determines the possibility of a disease on the basis of the biological information that is continuous in time series.

Note that the installation place of the holding device 1 is not limited to a residence of the subject, but may be a facility (for example, a nursing-care facility) other than a residence. In this case, the portable information processing terminals 2 of the respective facility users (the respective subjects) are attached to the holding devices 1, so that biological information about the respective facility users is detected via the portable information processing terminals 2 of the respective facility users. The holding device 1 may also be installed in a vehicle or the like that is used by the subject.

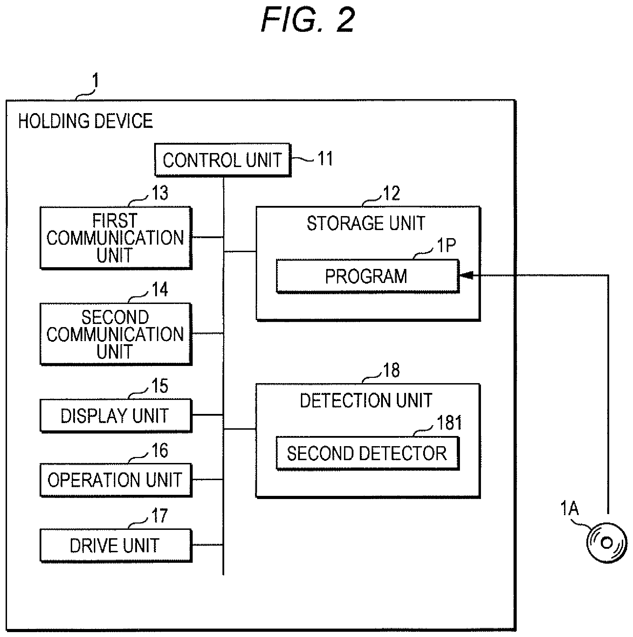

Fig. 2 is a block diagram illustrating the configuration of the holding device 1. The holding device 1 includes a control unit 11, a storage unit 12, a first communication unit 13, a second communication unit 14, a display unit 15, an operation unit 16, a drive unit 17, and the like.

The control unit 11 is an arithmetic processing unit that includes a central processing unit (CPU), a graphics processing unit (GPU), and the like. The control unit 11 executes various computer programs stored in a ROM or the storage unit 12 using a memory such as an internal read only memory (ROM) or random access memory (RAM), and controls operations of the respective components of the hardware described above, to perform a process of acquiring biological information. The control unit 11 may have functions such as a timer that measures an elapsed time since issuance of a measurement start instruction till issuance of a measurement end instruction, a counter that counts numbers, and a clock that outputs time and date information.

The storage unit 12 is a nonvolatile storage device such as a hard disk, an electrically erasable programmable ROM (EEPROM), or a flash memory. The storage unit 12 stores various kinds of computer programs and data. The computer programs stored in the storage unit 12 include a program 1P for causing a computer to perform a process related to acquisition of biological information. The data stored in the storage unit 12 includes a holding device ID of the holding device 1.

A computer program (a computer program products) to be stored into the storage unit 12 may be provided by a non-transitory recording medium 1A in which the computer program is recorded in a readable manner. The recording medium 1A is a portable memory such as a CD-ROM, a USB memory, or a secure digital (SD) card. The control unit 11 reads a desired computer program from the recording medium 1A using a reading device (not shown in the drawing), and stores the read computer program into the storage unit 12. Alternatively, the computer program may be provided by communication. The program 1P can be disposed at one site in a single computer, or be dispersed over a plurality of sites to be run on multiple computers interconnected by a communication network.

The first communication unit 13 includes a communication device that performs communication via a network N or according to a predetermined mobile communication standard. The control unit 11 transmits and receives various kinds of information to and from the information processing device 3 through the first communication unit 13.

The second communication unit 14 includes a communication device that performs communication by near field communication such as Bluetooth (registered trademark), WiFi (registered trademark), or Low Energy, for example. The control unit 11 transmits and receives various kinds of information to and from the portable information processing terminal 2 through the second communication unit 14. The second communication unit 14 may be a communication device (a connecting terminal) for communication, such as a universal serial bus (USB) (registered trademark). In this case, the holding device 1 also functions as a charger that transmits power to the portable information processing terminal 2 via the second communication unit 14.

The display unit 15 includes a display device such as a liquid crystal panel or an organic electro luminescence (EL) display, and an output device such as an LED lamp. The display unit 15 displays various kinds of information in accordance with an instruction from the control unit 11. The holding device 1 may include a sound output device for reproducing sound, such as a speaker.

The operation unit 16 is an interface that accepts a user's operation, and includes physical buttons, a changeover switch, a touch panel device with a built-in display, and the like, for example. The operation unit 16 accepts an input of an operation from the user, and sends a control signal corresponding to the operation contents to the control unit 11.

The drive unit 17 has a pan/tilt mechanism for rotationally driving the holding unit 101 in horizontal and vertical directions. The drive unit 17 may include an actuator, an angular velocity sensor, and the like, for example. The control unit 11 can rotate the holding unit 101 in horizontal and vertical directions (pan rotation and tilt rotation) via the drive unit 17. As the holding unit 101 rotates, the directivity of the first detector 271 of the portable information processing terminal 2 held by the holding unit 101 changes. The drive unit 17 may has either a pan mechanism or a tilt mechanism, and rotate the holding unit 101 only in either a horizontal or vertical direction.

The holding device 1 may further include a detection unit 18. The detection unit 18 includes a second detector 181 that detects biological information. The control unit 11 acquires the biological information detected by the detection unit 18. Note that the detection unit 18 is not necessarily included.

Fig. 3 is a block diagram illustrating the configuration of the portable information processing terminal 2. The portable information processing terminal 2 includes a control unit 21, a storage unit 22, a first communication unit 23, a second communication unit 24, a display unit 25, an operation unit 26, and a detection unit 27, which are similar to those of the holding device 1.

The control unit 21 is an arithmetic processing unit including a CPU, a GPU, and the like. The control unit 21 executes various computer programs stored in the ROM or the storage unit 22 using a memory such as an internal ROM or RAM, and controls operations of the respective components of the above hardware, to perform a process of detecting biological information.

The storage unit 22 includes a nonvolatile storage device such as a hard disk, an EEPROM, or a flash memory. The storage unit 22 stores various kinds of computer programs and data. The computer programs stored in the storage unit 22 include an application program for causing a computer to perform a process related to acquisition of biological information.

The first communication unit 23 includes a communication device that performs communication via the network N or according to a predetermined mobile communication standard. The second communication unit 24 includes a communication device that performs communication by the above-described near field communication.

The display unit 25 includes a display device such as a liquid crystal panel or an organic EL display. The operation unit 26 is an interface that accepts a user's operation, and includes physical buttons, a touch panel device with a built-in display, and the like, for example.

The detection unit 27 includes a plurality of first detectors 271 that detect biological information. As described above, the first detectors 271 may include a camera, a microphone, an infrared sensor, a millimeter-wave sensor, and the like, for example.

The camera is an imaging device that includes a lens, an imaging element, and the like, photoelectrically converts light incident through the lens with the imaging element, and outputs image data. From the image data, the shapes and motions related to the face, hands, and feet of the subject can be detected. The microphone is a sound collecting device that includes an amplifier, an analog/digital (A/D) converter, and the like, and outputs audio data. An utterance state of subject and utterance contents can be detected from the audio data.

The infrared sensor (laser sensor) is an infrared laser such as LiDAR, an infrared camera, or the like, for example. The LiDAR includes a light emitting element that irradiates the subject with infrared rays, and a light receiving element that receives the infrared rays that have been emitted toward the subject and been reflected by the subject. The LiDAR calculates the distance to the subject, on the basis of the reciprocating time until the return of the infrared rays emitted toward the subject and reflected by the subject. By doing so, the LiDAR outputs point cloud data (signal data) that is three-dimensional information about the subject. The point cloud data can be converted into two-dimensional infrared image data. The infrared camera is a camera that includes a lens that receives infrared rays reflected by a biological surface or clothes of the subject, and a CMOS image sensor. The infrared camera outputs infrared image data (signal data) as two-dimensional information about the subject. The infrared camera also measures infrared rays emitted from the body of the subject, converts the measured amount of infrared rays into a temperature, and outputs the skin temperature of the subject.

The millimeter-wave sensor includes a synthesizer that generates a millimeter-wave signal, a transmission antenna, a reception antenna, a mixer, and the like, and detects a heartbeat of the heart or pulsation of a blood vessel on the basis of displacement of the biological surface of the subject. On the basis of the distance to the subject obtained by the millimeter-wave sensor, it is possible to calculate variation of the distance to the displacement site where the biological surface of the subject is displaced. In other words, it is possible to calculate displacement of the biological surface, and detect pulsation of the heart or blood vessels, and respiration at the displacement site.

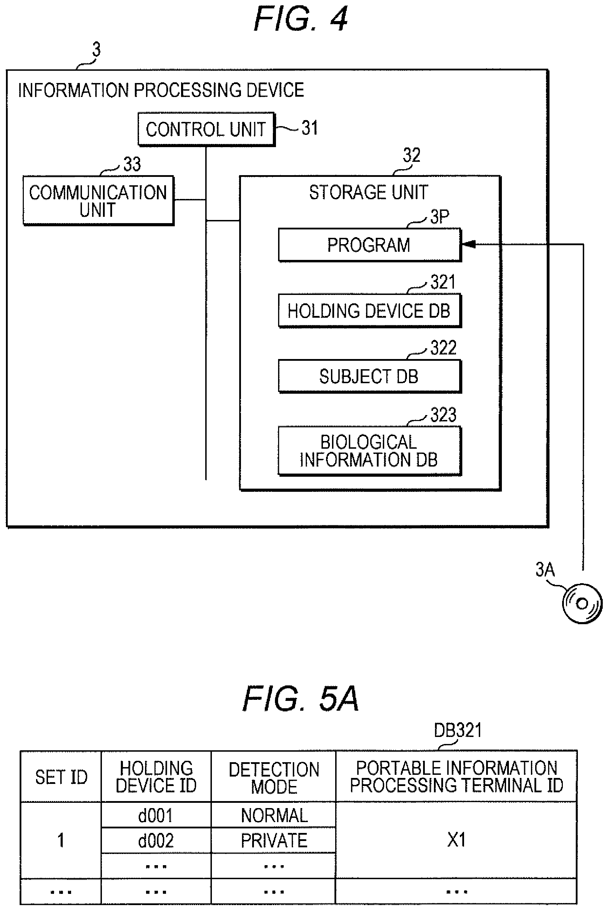

Fig. 4 is a block diagram illustrating the configuration of the information processing device 3. The information processing device 3 includes a control unit 31, a storage unit 32, and a communication unit 33.

The control unit 31 is an arithmetic processing unit including a CPU, a GPU, and the like. The control unit 31 executes various computer programs stored in the ROM or the storage unit 32 using a memory such as an internal ROM or RAM, and controls operations of the respective components of the above hardware, to perform a process related to acquisition of biological information and determination of the health condition. The control unit 31 may have functions such as a timer that measures an elapsed time since issuance of a measurement start instruction till issuance of a measurement end instruction, a counter that counts numbers, and a clock that outputs time and date information.

The storage unit 32 includes a nonvolatile storage device such as a hard disk, an EEPROM, or a flash memory. The storage unit 32 stores various kinds of computer programs and data. The computer programs stored in the storage unit 32 include a program 3P for causing a computer to perform a process related to acquisition of biological information and determination of the health condition. The data stored in the storage unit 32 includes a holding device database (DB) 321, a subject DB 322, and a biological information DB 323.

A computer program (a computer program products) to be stored into the storage unit 32 may be provided by a non-transitory recording medium 3A in which the computer program is recorded in a readable manner. The recording medium 3A is a portable memory such as a CD-ROM, a USB memory, or an SD card. The control unit 31 reads a desired computer program from the recording medium 3A using a reading device (not shown in the drawing), and stores the read computer program into the storage unit 32. Alternatively, the computer program may be provided by communication. The program 3P can be disposed at three sites in a single computer, or be dispersed over a plurality of sites to be run on multiple computers interconnected by a communication network.

The communication unit 33 includes a communication device that performs communication via the network N or according to a predetermined mobile communication standard. The control unit 31 transmits and receives various kinds of information to and from each device through the communication unit 33.

Figs. 5A to 5C are explanatory diagrams illustrating examples of record layouts in the holding device DB 321, the subject DB 322, and the biological information DB 323.

Fig. 5A is a diagram illustrating an example of the record layout in the holding device DB 321. The holding device DB 321 is a database that stores information about each holding device of a plurality of holding devices 1. The holding device DB 321 stores set IDs, holding device IDs, detection modes, portable information processing terminal IDs, and the like, which are associated with each other, for example.

A set ID is identification information for identifying a sales set in a case where a plurality of holding devices 1 is sold as one set. A holding device ID is identification information for identifying a holding device 1, and includes the device ID or the IP address of the holding device 1, for example. A detection mode is a detection mode set in a holding device 1, and includes "Normal" indicating a normal mode or "Private" indicating a private mode, for example. Detection modes will be described later in detail in other embodiments. A portable information processing terminal ID is identification information for identifying the portable information processing terminal 2 paired with a holding device 1, and includes the device ID, the IP address, or the like of the portable information processing terminal 2, for example. The information in the holding device DB 321 may be collected in accordance with an initial registration operation that is performed at the manufacturing stage of the holding devices 1 or after the sale of the holding devices 1.

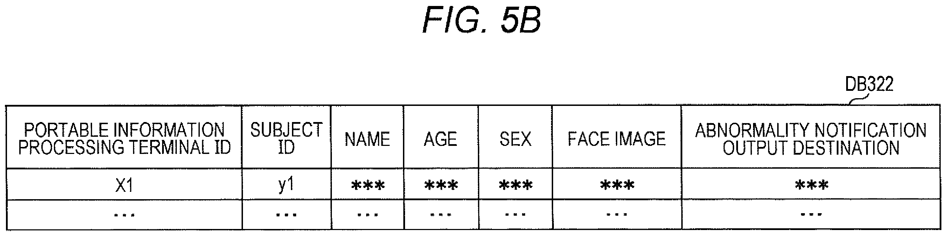

Fig. 5B is a diagram illustrating an example of the record layout in the subject DB 322. The subject DB 322 is a database that stores information about the subjects who use the holding devices 1. The subject DB 322 stores portable information processing terminal IDs, subject IDs, the name, age, sex, and a face image of each subject, abnormality notification output destinations, and the like, which are associated with each other, for example.

A portable information processing terminal ID is identification information for identifying a portable information processing terminal 2. A subject ID is identification information for identifying a subject as a biological information detection target using a portable information processing terminal 2. A face image is information for identifying a subject, and may include image data obtained by imaging the face of the subject. The information for identifying a subject is not necessarily a face image, but may be voice data, the height of the subject, or the like. An abnormality notification output destination is information about the output destination to which a notification is to be output in a case where the health condition of the subject is abnormal. Each abnormality notification output destination may include the IP address, a telephone number, an e-mail address, or the like of each corresponding portable information processing terminal 2 or communication terminal 4, for example.

In a case where a subject uses the information processing system S for the first time, for example, the information in the subject DB 322 is collected by receiving a registration operation from the subject via a registration screen provided on the information processing device 3. Note that the number of subjects to be monitored by one portable information processing terminal 2 may be one, or may be two or larger. In a case where a plurality of subjects is to be monitored, information regarding the plurality of subjects is associated with the one portable information processing terminal 2 and is then registered.

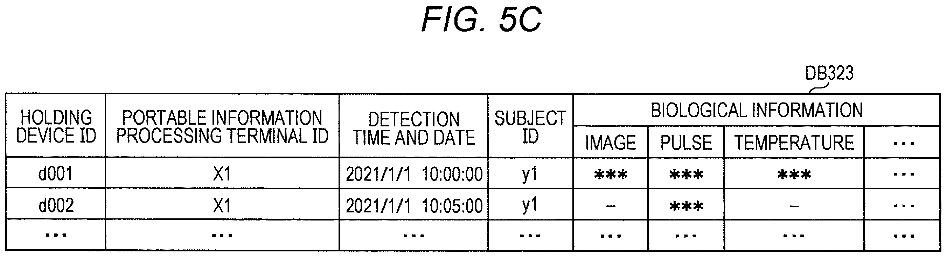

Fig. 5C is a diagram illustrating an example of the record layout in the biological information DB 323. The biological information DB 323 is a database that stores biological information about subjects. The biological information DB 323 stores holding device IDs, portable information processing terminal IDs, detection times and dates, subject IDs, biological information, and the like, which are associated with each other, for example.

A holding device ID and a portable information processing terminal ID are identification information about a holding device 1 and a portable information processing terminal 2 that have acquired biological information. A detection time and date is a time and date of detection of biological information. A subject ID is identification information about a subject as a biological information detection target. The biological information includes various kinds of biological information detected by the first detector 271. The biological information includes image data, heartbeat or pulse data, voice data, and the like, for example. The information in the biological information DB 323 is recorded each time information is acquired from a portable information processing terminal 2 via a holding device 1.

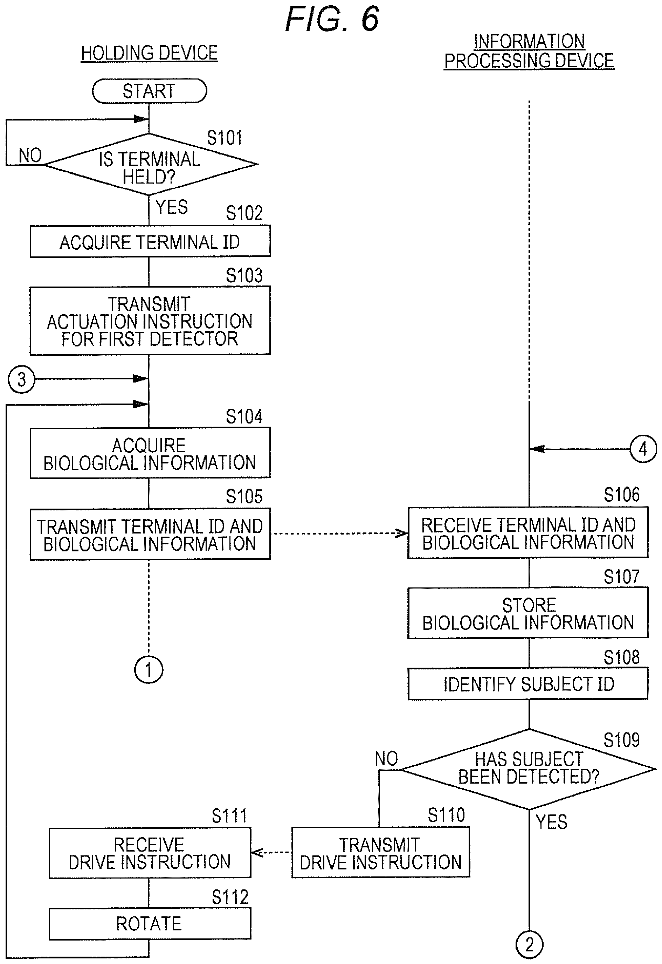

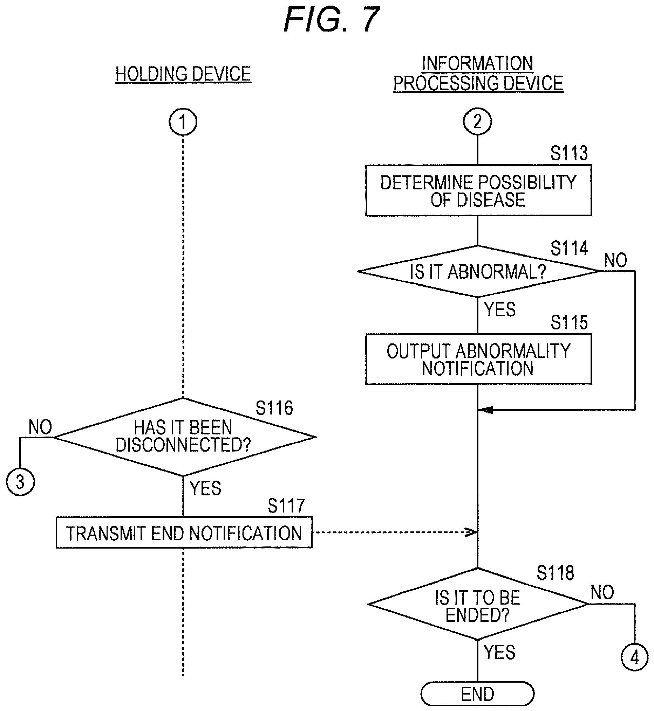

Figs. 6 and 7 are flowcharts illustrating information processing procedures according to the first embodiment. The process described below is performed by the control unit 11 according to the program IP stored in the storage unit 12 of the holding device 1, and is executed by the control unit 31 according to the program 3P stored in the storage unit 32 of the information processing device 3. The information processing system S may repeatedly perform the following process at predetermined or appropriate time intervals, for example.

The control unit 11 of the holding device 1 determines whether the portable information processing terminal 2 is held (step S101). The control unit 11 transmits a communication establishment request by communication using a predetermined communication standard, for example, receives a response signal from the portable information processing terminal 2, and establishes intercommunication, to determine that the portable information processing terminal 2 is set, or, in other words, the portable information processing terminal 2 is held. The control unit 11 may determine that the portable information processing terminal 2 is held, in a case where a detection device or the like provided in the holding unit 101 detects that the portable information processing terminal 2 is set to the holding unit 101. The control unit 11 may detect that the portable information processing terminal 2 is set to the holding unit 101 via a connecting terminal or the like.

Note that, in a case where the subject uses the holding device 1 for the first time, initial setting is performed so that an advance operation to pair the portable information processing terminal 2 with the holding device 1 enables the operation system (OS) of the portable information processing terminal 2 to accept a control instruction from the holding device 1.

If intercommunication has not been established, and it is determined that the holding unit 101 is not holding the portable information processing terminal 2 (step S101: NO), the control unit 11 returns the process to step S101, and waits until the portable information processing terminal 2 is set.

If intercommunication has been established, and it is determined that the holding unit 101 is holding the portable information processing terminal 2 (step S101: YES), the control unit 11 acquires the portable information processing terminal ID of the portable information processing terminal 2 with which intercommunication has been established (step S102).

The control unit 11 turns on the detection mode of the portable information processing terminal 2, and transmits an actuation instruction for the first detector 271 to the portable information processing terminal 2 (step S103). The control unit 11 outputs, to the portable information processing terminal 2, an actuation instruction for actuating the first detector 271 necessary for acquiring biological information, and causes the first detector 271 to perform detection. Since the pairing has been completed between the portable information processing terminal 2 and the holding device 1, there is no need to start an application or the like, and, after the portable information processing terminal 2 is simply set to the holding device 1, the control unit 11 can perform control related to actuation of the first detector 271. Accepting the control instruction from the holding device 1, the portable information processing terminal 2 detects biological information with the first detector 271, and transmits the detected biological information to the holding device 1. The biological information detection and transmission processes by the first detector 271 may be performed in the background.

The control unit 11 acquires the biological information transmitted from the portable information processing terminal 2 (step S104). The biological information may include a plurality of pieces of biological information detected by a plurality of first detectors 271. The control unit 11 transmits, to the information processing device 3, the portable information processing terminal ID of and the biological information about the portable information processing terminal 2, and the holding device ID of the holding device 1 (step S105).

The control unit 31 of the information processing device 3 receives the portable information processing terminal ID, the biological information, and the holding device ID (step S106). The control unit 31 stores the acquired portable information processing terminal ID, biological information, and holding device ID into the biological information DB 323 (step S107). On the basis of the information stored in the subject DB 322, the control unit 31 identifies the subject ID associated with the portable information processing terminal ID (step S108).

The control unit 31 performs a process of analyzing the acquired biological information, and determines whether the subject as the detection target has been detected (step S109). For example, the control unit 31 performs pattern matching on the image data in the acquired biological information, and determines whether a biological object (a person) is extracted from the image data.

If it is determined that the subject has not been detected because any biological object has not been extracted from the image data (step S109: NO), the control unit 31 transmits a drive instruction to the holding device 1 (step S110). The drive instruction includes information for rotating the holding unit 101 so as to change the directivity of the first detector 271 in accordance with the position of the subject. If part of a biological object is included in the image data, the control unit 31 determines the pan/tilt angle of the holding unit 101 so that the biological object is located at the center of the image data, on the basis of the position coordinates of the biological object in the image data. In a case where any biological object is not included in the image data, the control unit 31 may determine the rotation angle of the holding unit 101, according to a predetermined rule.

If it is determined that the subject has been detected because a biological object has been extracted from the image data (step S109: YES), the control unit 31 skips the process of transmitting the drive instruction, and moves the process on to step S113.

The control unit 11 of the holding device 1 receives the drive instruction (step Sill). The control unit 11 controls the drive unit 17 in accordance with the received drive instruction, and rotates the holding unit 101 in the direction of the subject (step S112). The control unit 11 changes the directivity of the first detector 271, and then returns the process to step S104.

On the basis of the acquired biological information, the control unit 31 of the information processing device 3 determines the possibility of a disease of the subject, which is the possibility of an abnormality of blood vessels or the heart, according to a predetermined determination rule (step S113).

The control unit 31 performs shape analysis on a displacement portion on the basis of a detection value supplied from the camera, the infrared sensor, or the millimeter-wave sensor, for example, to calculate a temporal change in the heartbeat, the pulse rate, the heart rate, the pulse rhythm, the magnitude of pulse fluctuations, the peak point of time of the heartbeat, or the like. The temporal change in the heartbeat is, in other words, a temporal change in the amount of displacement of the biological surface at a displacement site. The pulse rate is the number of pulsations of the artery per minute, for example. The heart rate is the number of pulsations of the heart per minute, for example. The pulse rhythm is a numerical value indicating a pulsation cycle or regularity of pulsation cycles at the displacement portion, for example. The magnitude of pulse fluctuations is the amplitude of displacement of the biological surface at the displacement site. The peak point of time of the heartbeat is the point of time at which the amount of displacement at the displacement site is the largest. Note that the point of time at which the amount of displacement is the smallest may be detected.

The control unit 31 determines whether the above calculated temporal change in the heartbeat, the pulse rate, the heart rate, the pulse rhythm, the magnitude of pulse fluctuations, the peak point of time of the heartbeat, or the like, or the amount of change (difference) of calculated temporal change in the heartbeat, the pulse rate, the heart rate, the pulse rhythm, the magnitude of pulse fluctuations, the peak point of time of the heartbeat, or the like is equal to or greater than a predetermined threshold value. By doing so, the control unit 31 determines whether there is a possibility of cerebral infarction, cerebral hemorrhage, atrial cells, foot infarction, or varicose vein. For example, the control unit 31 determines that there is a possibility of a disease in a case where the detection result (calculation result) is equal to or greater than a predetermined threshold, but determines that there is no possibility of a disease in a case where the detection result is smaller than the predetermined threshold. As an example, in a case where the pulse fluctuations in the dorsal artery of foot are smaller than a predetermined threshold on the basis of the magnitude of the pulse fluctuations in the dorsal artery of foot, the control unit 31 determines that there is a possibility of critical limb ischemia (foot infarction), or, in other words, there is an abnormality.

The threshold serving as the criterion may be determined as appropriate in accordance with attribute information such as age and sex of the subject, or may be individually determined on the basis of the past biological information or the like about the subject. In a case where the subject has a chronic disease or a preexisting condition, an abnormality threshold is preferably set for each subject by medical personnel such as a medical doctor, in accordance with chronic diseases and preexisting conditions. For example, in a case where the subject has a heart disease, a threshold for pulse fluctuations is set to a smaller value than usual, so that an abnormality can be detected at an earlier stage. The threshold serving as the criterion may be changed as needed, depending on the state of the subject's chronic disease or preexisting condition.

For example, the control unit 31 may perform a motion analysis on the target sides on the basis of detection values supplied from the cameral, the infrared sensor, or the millimeter-wave sensor, for example, to calculate the amounts of motion at the target sites in the right side and the left side of the body of the subject. The control unit 31 determines whether the calculated amounts of motion or the difference in the amount of motion between the right side and the left side of the body is equal to or larger than a predetermined threshold, to determine whether there is a possibility of cerebral infarction or cerebral hemorrhage. As an example, on the basis of image data of a plurality of frames related to the same subject acquired in time series, the control unit 31 compares the coordinates of the left hand and the right hand in the previous frame with the coordinates of the left hand and the right hand in the current frame, and acquires the moving velocity calculated from the movement distance and the detection times. In a case where the right-left difference between the differences obtained by comparing the acquired movement velocities of the left hand and the right hand with reference movement velocities of the left hand and the right hand stored in advance is equal to or larger than a predetermined threshold, the control unit 31 determines that there is a possibility of cerebral infarction, or, in other words, there is an abnormality.

The control unit 31 may perform a shape analysis on a displacement site or a motion analysis on the target site on the basis of detection values supplied from the camera, the infrared sensor, or the millimeter-wave sensor, for example, to calculate the shapes, thicknesses, swellings, swelling sizes, and the like of the face, the hands, the feet, the jugular veins, and the lower limb artery. The control unit 31 determines whether the calculation result is equal to or greater than a predetermined threshold, to determine whether there is a possibility of heart failure, varicose vein, or deep vein thrombus. As an example, in a case where the swelling size of the jugular veins is equal to or larger than a threshold on the basis of the swelling size of the jugular veins, the control unit 31 determines that there is a possibility of jugular venous distention, and there is a possibility of heart failure, or, in other words, there is an abnormality.

The control unit 31 may calculate the temperature or the amount of change in temperature of the target region on the basis of detection values from the infrared sensor, or may calculate the color tone or the amount of change in color tone of the target site on the basis of detection values from the camera. On the basis of the calculated temperature and color tone, the control unit 31 determines the presence or absence of a possibility of critical limb ischemia (foot infarction) and deep vein thrombus.

In the process described above, the control unit 31 does not necessarily determine that there is a possibility of a disease in a case where a detection result is equal to or greater than a predetermined threshold, but may determine that there is a possibility of a disease in a case where a detection result is smaller than the predetermined threshold, depending on the contents of the acquired biological information and the threshold. Further, the above method for determining a possibility of a disease in accordance with biological information is an example and is not limited to any particular method.

The control unit 31 determines whether the determination result is abnormal (step S114). If it is determined that there is no abnormality without a possibility of a disease (step S114: NO), the control unit 31 skips the process of outputting an abnormality, and moves the process on to step S118.

If it is determined that there is an abnormality due to a possibility of a disease (step S114: YES), the control unit 31 outputs an abnormality notification indicating the abnormality of the subject (step S115). On the basis of the information stored in the subject DB 322, the control unit 31 identifies the abnormality notification output destination associated with the subject ID for which the abnormality has been detected, and outputs an abnormality notification to the identified abnormality notification output destination. The control unit 31 outputs the abnormality notification to the communication terminal 4 of medical personnel, for example. The control unit 31 may output the abnormality notification to the portable information processing terminal 2 of the subject, the display unit 15 of the holding device 1, or the like. The abnormality notification may include the name of the subject, the detection value related to the abnormality, the detection time and date, and the like, for example. The abnormality notification may be output with a lamp turned on to indicate an abnormality, audio data, or the like.

The control unit 11 of the holding device 1 determines whether the portable information processing terminal 2 has been detached (step S116). For example, if it is determined that the portable information processing terminal 2 has not been detached from the holding unit 101 because the intercommunication with the portable information processing terminal 2 has not been cut off (step S116: NO), the control unit 11 returns the process to step S104, and continues the acquisition of biological information.

If it is determined that the portable information processing terminal 2 has been detached from the holding unit 101 due to cut-off of the intercommunication with the portable information processing terminal 2 (step S116: YES), the control unit 11 notifies the information processing device 3 of an end (step S117).

The control unit 31 of the information processing device 3 determines whether to end the acquisition of biological information (step S118). If it is determined that the process is not to end because an end notification has not been received (step S118: NO), the control unit 11 returns the process to step S106, and continues the acquisition of biological information. If it is determined to end the process because an end notification has been received (step S118: YES), the control unit 11 ends the series of processes.

In step S109 described above, the control unit 31 may determine whether the subject corresponding to the subject ID has been detected. The control unit 31 extracts a face feature quantity from a face image of the subject corresponding to the subject ID in advance. The control unit 31 then extracts the face feature quantity of the subject from the image data in the biological information acquired in step S107, and performs pattern matching with a face feature quantity of the subject registered in advance. By doing so, the control unit 31 identifies the subject included in the image. The control unit 31 may identify the subject included in the detected biological information, on the basis of other information for identifying the subject, such as voice data, for example. As a result, even in a case where there is a plurality of persons in the room, it is possible to correctly identify the subject, and acquire biological information related to the identified subject. Thus, the subject can be monitored in a preferred manner.

Although an example in which the control unit 11 of the holding device 1 and the control unit 31 of the information processing device 3 perform the respective processes has been described above, each of the above processes described above is not necessarily performed in this manner. The control unit 11 of the holding device 1 and the control unit 31 of the information processing device 3 may perform processes in cooperation with each other. The holding device 1 may also function as the information processing device 3.

Further, part of each process described above may be performed by the control unit 21 of the portable information processing terminal 2. In this case, the portable information processing terminal 2 may store, in the storage unit 22, an application program (not shown) for performing a process related to acquisition of biological information, and the control unit 21 may perform the process according to the application program. For example, the control unit 21 of the portable information processing terminal 2 may transmit detected biological information directly to the information processing device 3. The control unit 21 may also perform an analysis process as to whether the subject is included in the biological information.

According to the present embodiment, biological information about the subject can be detected in a preferred manner, and a possibility of a disease of the subject can be determined on the basis of the detected biological information. As a plurality of holding devices 1 is provided within the movement range of the subject, it is possible to reduce the non-detection regions and the non-detection times for biological information, and continuously acquire biological information. A possibility of a disease can be accurately determined on the basis of continuous biological information, and failure to detect an abnormality can be reduced. As biological information is continuously acquired in daily life, the progress of a disease and a possibility of onset of a disease can be indicated at an early stage.

The information processing system S acquires biological information using the first detector 271 provided as standard in the portable information processing terminal 2 such as a smartphone, so that the cost of the holding device 1 can be made lower than in a case where a sensor device is provided in the holding device 1. Thus, the initial cost can be lowered, and the holding device 1 can be easily introduced. Further, because of high cost performance, a plurality of holding devices 1 can be easily sold as a set. As a result, multi-point detection by a large number of holding devices 1 becomes possible, and biological information can be acquired in a preferred manner. As the holding device 1 functions as a temporary stand for the portable information processing terminal 2, the subject can attach the portable information processing terminal 2 to the holding device 1 with a natural action, without any burden on the subject. Thus, the holding device 1 does not limit installation places, and can be suitably installed in various places in daily life where sensing devices have not been actively installed in the past.

### Second Embodiment

In a second embodiment, the first detector 271 is actuated in accordance with the detection mode of the holding device 1. In the description below, differences from the first embodiment are mainly described. The same components as those of the first embodiment are denoted by the same reference numerals as those used in the first embodiment, and detailed explanation of the same components as those of the first embodiment will not be repeated.

As for holding devices 1 of the second embodiment, a detection mode is set beforehand in each holding device 1. The detection mode is a detection mode to be applied at the time of detection of biological information, and includes a normal mode and a private mode, for example. In the normal mode, a plurality of first detectors 271 included in a portable information processing terminal 2 is actuated without limitation. In the private mode, of a plurality of first detectors 271 included in the portable information processing terminal 2, first detectors 271 other than the first detectors 271 that detect biological information regarding privacy, such as a camera and a microphone, are actuated, for example. That is, in the private mode, only the first detectors 271 that do not detect biological information regarding privacy are actuated. For example, in a case where the holding device 1 is installed in a toilet, a dressing room, a bathroom, or the like, it is preferable to adopt a private mode for detecting only biological information excluding images and voice of the subject. In the private mode, for example, the heart rate, pulse, respiration, body temperature, or the like detected by an infrared sensor or a millimeter-wave sensor is collected, for example.

The holding device 1 stores the detection mode beforehand in the storage unit 12. The detection mode may be set for each holding device 1 in advance at the stage of manufacture of the holding devices 1, for example. The subject appropriately selects and purchases holding devices 1 in which the normal mode or the private mode has been set in accordance with the number of rooms to be installed and the details of the rooms. The holding devices 1 may be sold as a set product that is a combination of a predetermined number of devices in the normal mode and a predetermined number of devices in the private mode.

The detection mode is not necessarily set beforehand in the holding device 1, but may be set or changed at a desired timing via an operation unit 16 (for example, a detection mode setting button or the like) provided in the holding device 1, for example. The detection mode may be set or changed by accepting an input operation from the subject via a setting screen provided by the information processing device 3 when the subject uses the holding device 1, for example. The holding device 1 acquires the set or changed detection mode through the operation unit 16 or the information processing device 3, and stores the detection mode into the storage unit 12.

The detection mode may further include a customized mode. In the customized mode, to detect only biological information to meet a request of the subject, only a first detector 271 that detects biological information to meet the request of the subject is selectively actuated. For example, the control unit 31 of the information processing device 3 receives the biological information requested by the subject, the determination item requested by the subject, the disease requested to be determined, and the like via a setting screen provided on the information processing device 3. By doing so, the control unit 31 identifies the type of the first detector 271 to be actuated. The control unit 31 transmits the identified first detector 271 to the holding device 1. The holding device 1 receives the first detector 271 transmitted from the information processing device 3, to accept the setting of the customized mode. The acceptance of the detection mode and the setting of each item may be performed via an application program of the portable information processing terminal 2.

Note that the customized mode is not necessarily realized by selectively actuating only a predetermined first detector 271, but may be realized by acquiring only predetermined biological information from the biological information detected by a plurality of first detectors 271 and determining a possibility of a disease using only the acquired predetermined biological information.

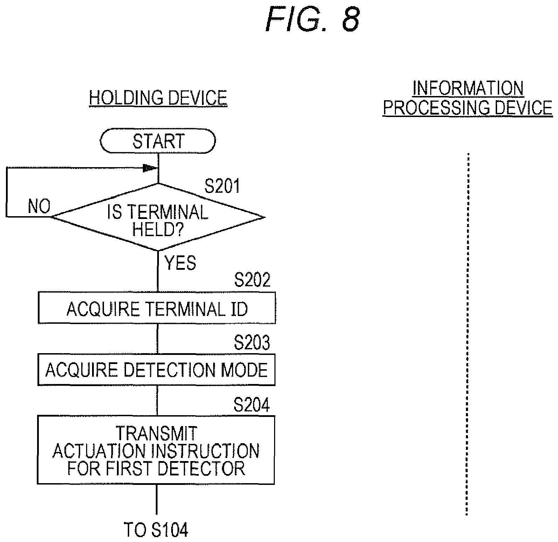

Fig. 8 is a flowchart illustrating information processing procedures according to the second embodiment.

The control unit 11 of the holding device 1 determines whether the portable information processing terminal 2 is held (step S201). If intercommunication has not been established, and it is determined that the holding unit 101 is not holding the portable information processing terminal 2 (step S201: NO), the control unit 11 returns the process to step S201, and waits until the portable information processing terminal 2 is set.

If intercommunication has been established, and it is determined that the holding unit 101 is holding the portable information processing terminal 2 (step S201: YES), the control unit 11 acquires the portable information processing terminal ID of the portable information processing terminal 2 with which intercommunication has been established (step S202).

The control unit 11 acquires a detection mode, on the basis of the information stored in the storage unit 12 (step S203). The control unit 11 may transmit an inquiry to the information processing device 3, and receive a response from the information processing device 3, to acquire a detection mode. The control unit 31 of the information processing device 3 identifies the detection mode associated with the holding device ID of the holding device 1 from which the inquiry has been accepted, on the basis of the information stored in the holding device DB 321. The control unit 31 then transmits the identified detection mode to the holding device 1 that is the inquirer.

The control unit 11 turns on the detection mode of the portable information processing terminal 2 in accordance with the acquired detection mode, and transmits, to the portable information processing terminal 2, an actuation instruction for the first detector 271 compatible with the detection mode (step S204). For example, in a case where the detection mode is the private mode, the control unit 11 transmits an actuation instruction to actuate only the first detectors 271 that do not detect biological information regarding privacy, excluding cameras, microphones, and the like. Thereafter, the information processing system S performs the process in steps S104 to S118 shown in Figs. 6 and 7, to perform acquisition of the biological information detected by the first detector 271 compatible with the detection mode, and a process of determining a possibility of a disease.

In the process described above, the control unit 11 preferably displays the acquired detection mode in an identifiable manner. For example, in accordance with each detection mode, the control unit 11 displays the detection mode in an identifiable manner by turning on an LED lamp in the display color associated with the detection mode.

According to the present embodiment, it is possible to monitor the subject in a preferred manner, while protecting the privacy of the subject. As a detection mode is set in the holding device 1 in advance, the operation burden on the subject can be reduced. Further, as a detection mode is configured to be set or changed as needed, the degree of freedom in use of the holding device 1 is increased, and the holding device 1 can be adopted in various usage modes.

### Third Embodiment

In a third embodiment, a detection mode is turned on in accordance with the illuminance in the surrounding of the holding device 1 or time. In the description below, differences from the first embodiment are mainly described. The same components as those of the first embodiment are denoted by the same reference numerals as those used in the first embodiment, and detailed explanation of the same components as those of the first embodiment will not be repeated.

A holding device 1 of the third embodiment determines whether to start a detection mode in accordance with the surrounding illuminance or time. The holding device 1 starts a detection mode, in a case where the surroundings of the holding device 1 are dark and the illuminance has become lower. For example, in a case where the subject uses the holding device 1 as a stand for a portable information processing terminal 2 and is viewing a moving image using the portable information processing terminal 2, the holding device 1 starts a detection mode at a time point at which the viewing of the moving image comes to an end and the surrounding illuminance becomes lower.

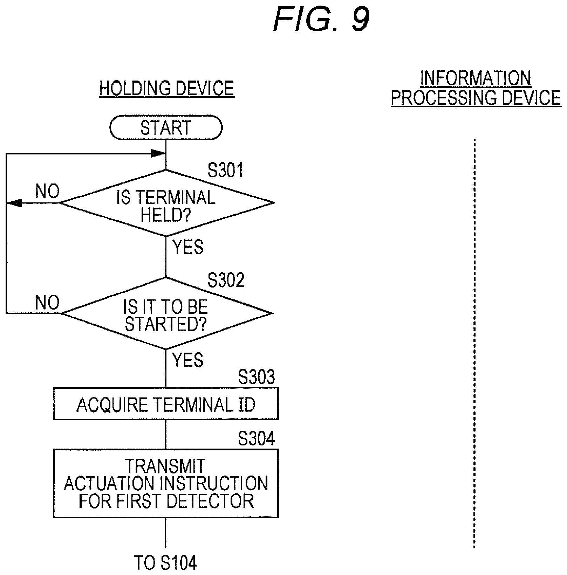

Fig. 9 is a flowchart illustrating information processing procedures according to the third embodiment.

The control unit 11 of the holding device 1 determines whether the portable information processing terminal 2 is held (step S301). If intercommunication has not been established, and it is determined that the holding unit 101 is not holding the portable information processing terminal 2 (step S301: NO), the control unit 11 returns the process to step S301, and waits until the portable information processing terminal 2 is set.

If intercommunication has been established, and it is determined that the holding unit 101 is holding the portable information processing terminal 2 (step S301: YES), the control unit 11 determines whether to start a detection mode (step S302) . The control unit 11 acquires the surrounding illuminance, and determines whether the acquired illuminance is lower than a preset threshold, to determine whether to start a detection mode. In a case where the acquired illuminance is lower than the preset threshold, the control unit 11 determines to start a detection mode. In a case where the acquired illuminance is equal to or higher than the preset threshold, the control unit 11 determines not to start a detection mode.

The control unit 11 may acquire the illuminance through a camera included among the first detectors 271 of the portable information processing terminal 2. The camera detects exposure, to detect the illuminance around the portable information processing terminal 2 held by the holding device 1. In a case where the first detectors 271 of the portable information processing terminal 2 include an illuminance sensor, the control unit 11 may receive a detection value obtained by the illuminance sensor from the portable information processing terminal 2, to acquire data regarding the illuminance.

In step S302, the control unit 11 may acquire the current time, and determine whether the acquired current time is within a preset operating time range, to determine whether to start a detection mode. In a case where the current time is within the preset operating time range, the control unit 11 determines to start a detection mode. In a case where the current time is out of the preset operating time range, the control unit 11 determines not to start a detection mode.

If it is determined not to start a detection mode (step S302: NO), the control unit 11 returns the process to step S301. If it is determined to start a detection mode (step S302: YES), the control unit 11 acquires the portable information processing terminal ID of the portable information processing terminal 2 (step S303).

The control unit 11 turns on the detection mode of the portable information processing terminal 2, and transmits an actuation instruction for first detector 271 to the portable information processing terminal 2 (step S304). As a result, acquisition of biological information is started. Thereafter, the information processing system S performs the process in steps S104 to S118 shown in Figs. 6 and 7, to perform acquisition of the biological information detected by the first detector 271, and a process of determining a possibility of a disease.

According to the present embodiment, the timing to perform acquisition of biological information can be adjusted. Thus, the functions of the portable information processing terminal 2 can be efficiently utilized, while the processing load on the portable information processing terminal 2 is reduced.

### Fourth Embodiment

In a fourth embodiment, biological information is detected with a holding device 1 or a detector installed in a residence. In the description below, differences from the first embodiment are mainly described. The same components as those of the first embodiment are denoted by the same reference numerals as those used in the first embodiment, and detailed explanation of the same components as those of the first embodiment will not be repeated.

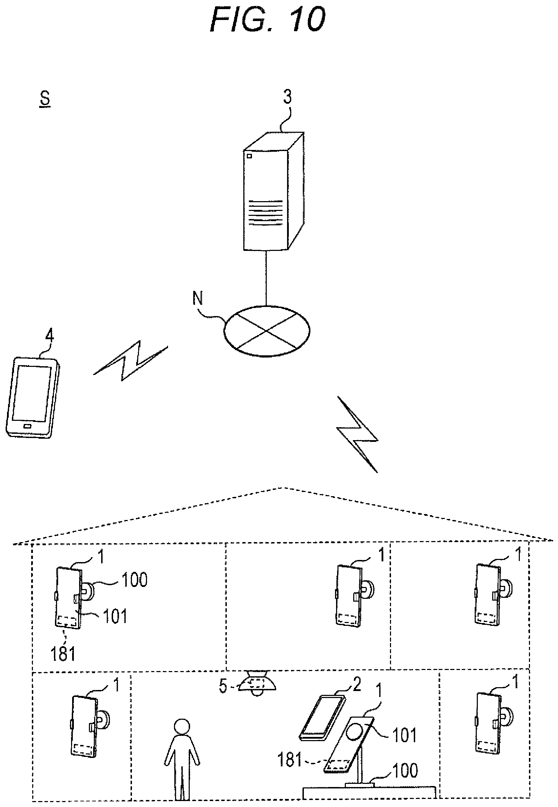

Fig. 10 is a schematic diagram of an information processing system S according to the fourth embodiment. In the information processing system S according to the fourth embodiment, in addition to the first detectors 271 of the portable information processing terminal 2, a detector that detects biological information is provided in a device (equipment) other than the portable information processing terminal 2.

As illustrated in Fig. 10, a holding device 1 of the fourth embodiment has a second detector 181 that detects biological information. Further, in the fourth embodiment, a third detector 5 is provided in a residence, a facility, or the like where the holding device 1 is installed. The third detector 5 is provided in a lighting device installed on the ceiling of a room, for example. The third detector 5 has a communication function, and transmits detected biological information to the holding device 1.

The second detector 181 included in the holding device 1 is preferably a detector (a sensor device) that detects biological information of a different type from the biological information to be detected by the first detector 271 of the portable information processing terminal 2. For example, the second detector 181 may be a sensor device that is not a camera that is usually provided in the portable information processing terminal 2. The holding device 1 may include a plurality of second detectors 181.

The third detector 5 may be a detector that detects biological information of the same type as that detected by the first detector 271 and the second detector 181, or may be a detector that detects biological information of a different type from that detected by the first detector 271 and the second detector 181. In a case where the third detector 5 detects biological information of the same type as that detected by the first detector 271 and the second detector 181, the third detector 5 is preferably provided at a site where biological information is detected from an orientation direction different from those with the first detector 271 and the second detector 181. A plurality of third detectors 5 may be provided in a residence, a facility, or the like.

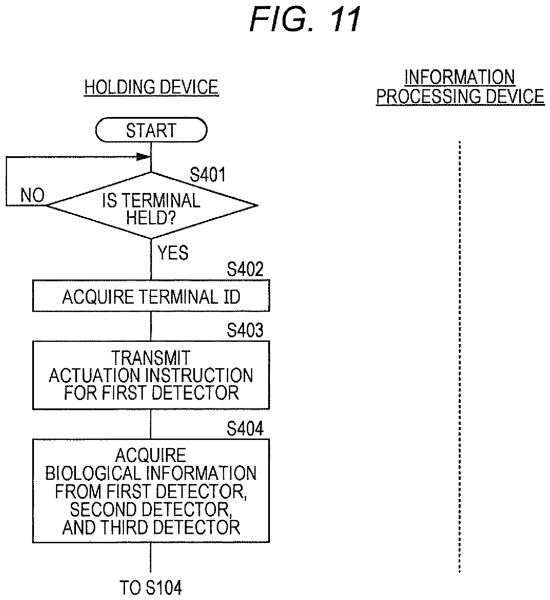

Fig. 11 is a flowchart illustrating information processing procedures according to the fourth embodiment.

The control unit 11 of the holding device 1 determines whether the portable information processing terminal 2 is held (step S401). If intercommunication has not been established, and it is determined that the holding unit 101 is not holding the portable information processing terminal 2 (step S401: NO), the control unit 11 returns the process to step S401, and waits until the portable information processing terminal 2 is set.

If intercommunication has been established, and it is determined that the holding unit 101 is holding the portable information processing terminal 2 (step S401: YES), the control unit 11 acquires the portable information processing terminal ID of the portable information processing terminal 2 (step S402).

The control unit 11 turns on the detection mode of the portable information processing terminal 2, and transmits an actuation instruction for the first detector 271 to the portable information processing terminal 2 (step S403). The first detector 271 of the portable information processing terminal 2 detects biological information.

The control unit 11 acquires biological information detected by each of the first detector 271, the second detector 181, and the third detector 5 (step S404). Specifically, the control unit 11 receives biological information transmitted from the portable information processing terminal 2, to acquire the biological information detected by the first detector 271. The control unit 11 receives biological information output from the detection unit 18 of the holding device 1, to acquire the biological information detected by the second detector 181. The control unit 11 receives biological information transmitted from the third detector 5, to acquire the biological information detected by the third detector 5. Thereafter, the information processing system S performs the process in steps S104 to S118 shown in Figs. 6 and 7, to perform acquisition of the biological information detected by the first detector 271, the second detector 181, and the third detector 5, and a process of determining a possibility of a disease. The information processing device 3 comprehensively determines a possibility of a disease, on the basis of a plurality of pieces of biological information detected by the first detector 271, the second detector 181, and the third detector 5.

The control unit 11 does not necessarily acquire the respective pieces of the biological information detected by the first detector 271, the second detector 181, and the third detector 5 at the same timing, but may acquire the biological information detected by each detector at any appropriate timing. Also, the third detector 5 may transmit the acquired biological information to the information processing device 3. Note that only either the second detector 181 or the third detector 5 may be provided.

According to the present embodiment, pieces of data are associated with each other and are complemented on the basis of a larger amount of biological information. Thus, a possibility of a disease can be determined more accurately. The first detector 271 of the portable information processing terminal 2 is utilized, and biological information that is difficult to be detected by the first detector 271 of the portable information processing terminal 2 is acquired from another detector, which is the second detector 181 or the third detector 5. Thus, it is possible to efficiently and effectively monitor the subject.

Regarding the respective embodiments described above, the following supplementary notes are further disclosed herein.

### (Note 1)

An information processing system including:
a holding device including a holding unit that holds a portable information processing terminal, and a communication unit that communicates with the portable information processing terminal, the portable information processing terminal including a first detector that detects biological information; and
a control unit that performs control for acquiring the biological information, in which,
when the portable information processing terminal is held by the holding unit, and communication between the portable information processing terminal and the holding device has been established via the communication unit, the control unit acquires the biological information detected by the first detector.

### (Note 2)

The information processing system according to Note 1, in which
a possibility of a disease is determined on the basis of the acquired biological information.

### (Note 3)

The information processing system according to Note 2, in which
a plurality of the holding devices is provided, and
the possibility of a disease is determined on the basis of a plurality of pieces of the biological information acquired when the portable information processing terminal is held by each holding device of the plurality of the holding devices.

### (Note 4)

The information processing system according to Note 2 or 3, in which
the biological information includes an image of a face, a hand, or a foot, a heart rate, a pulse, respiration, or a body temperature, and
the possibility of a disease related to an abnormality of a heart or a blood vessel is determined on the basis of a color, a shape, a motion, or a right-left difference of the face, the hand, or the foot, a respiratory rate, the body temperature, the heart rate or a pulse rate, the heart rate or a pulse rhythm, or a magnitude of a fluctuation of the heart rate or the pulse in the biological information.

### (Note 5)

The information processing system according to any one of Notes 1 to 4, in which
the holding unit rotatably holds the portable information processing terminal, and
the portable information processing terminal held by the holding unit is rotated, to change directivity of the first detector in accordance with a position of a biological object.

### (Note 6)

The information processing system according to any one of Notes 1 to 5, in which
the control unit acquires biological information different from the biological information detected by the first detector, the biological information being detected by a second detector that is provided in a device different from the portable information processing terminal and detects the biological information.

### (Note 7)

The information processing system according to Note 6, in which
the holding device includes the second detector that detects biological information of a different type from the biological information detected by the first detector included in the portable information processing terminal.

### (Note 8)

The information processing system according to any one of Notes 1 to 7, in which
the portable information processing terminal includes a plurality of the first detectors including a camera, and
the control unit acquires the biological information in one of a first acquisition mode in which the biological information detected by the plurality of the first detectors including the camera is acquired, or a second acquisition mode in which the biological information detected by the plurality of the first detectors excluding the camera is acquired, depending on the holding device or a position at which the holding device is installed.

### (Note 9)

The information processing system according to Note 8, in which
one of the first acquisition mode or the second acquisition mode is set in the holding device.

### (Note 10)

The information processing system according to any one of Notes 1 to 9, in which
the control unit starts acquiring the biological information, in accordance with one of illuminance around the holding device or time.

### (Note 11)

A program for causing a computer to perform a process of
acquiring biological information detected by a first detector, when a portable information processing terminal including the first detector that detects the biological information is held by a holding device including a holding unit that holds the portable information processing terminal and a communication unit that communicates with the portable information processing terminal, and communication between the portable information processing terminal and the holding device has been established via the communication unit.

### (Note 12)

An information processing method implemented by a computer to perform a process of
acquiring biological information detected by a first detector, when a portable information processing terminal including the first detector that detects the biological information is held by a holding device including a holding unit that holds the portable information processing terminal and a communication unit that communicates with the portable information processing terminal, and communication between the portable information processing terminal and the holding device has been established via the communication unit.

### Reference Signs List

- S: Information processing system
- 1: Holding device
- 101: Holding unit
- 11: Control unit
- 12: Storage unit
- 13: First communication unit
- 14: Second communication unit (communication unit)
- 15: Display unit
- 16: Operation unit
- 17: Drive unit
- 18: Detection unit
- 181: Second detector
- 1P: Program
- 1A: Recording medium
- 2: Portable information processing terminal
- 21: Control unit
- 22: Storage unit
- 23: First communication unit
- 24: Second communication unit
- 25: Display unit
- 26: Operation unit
- 27: Detection unit
- 271: First detector
- 3: Information processing device
- 31: Control unit
- 32: Storage unit
- 33: Communication unit
- 3P: Program
- 3A: Recording medium
- 4: Communication terminal
- 5: Third detector

## Claims

1. An information processing system comprising:
a holding device including a holding unit that holds a portable information processing terminal, and a communication unit that communicates with the portable information processing terminal, the portable information processing terminal including a first detector that detects biological information; and
a control unit that performs control for acquiring the biological information, wherein,
when the portable information processing terminal is held by the holding unit, and communication between the portable information processing terminal and the holding device has been established via the communication unit, the control unit acquires the biological information detected by the first detector.

2. The information processing system according to claim 1, wherein
a possibility of a disease is determined on a basis of the acquired biological information.

3. The information processing system according to claim 2, wherein
the holding device includes a plurality of holding devices, and
the possibility of a disease is determined on a basis of a plurality of pieces of the biological information acquired when the portable information processing terminal is held by each holding device of the plurality of holding devices.

4. The information processing system according to claim 2 or 3, wherein
the biological information includes one of an image of a face, a hand, or a foot, a heart rate, a pulse, respiration, or a body temperature, and
the possibility of a disease related to an abnormality of a heart or a blood vessel is determined on a basis of one of a color, a shape, a motion, or a right-left difference in the face, the hand, or the foot, a respiratory rate, the body temperature, the heart rate or a pulse rate, the heart rate or a pulse rhythm, or a magnitude of a fluctuation of the heart rate or the pulse in the biological information.

5. The information processing system according to claim 1 or 2, wherein
the holding unit rotatably holds the portable information processing terminal, and
the portable information processing terminal held by the holding unit is rotated, to change directivity of the first detector in accordance with a position of a biological object.

6. The information processing system according to claim 1 or 2, wherein
the control unit acquires biological information different from the biological information detected by the first detector, the biological information being detected by a second detector that is provided in a device different from the portable information processing terminal and detects the biological information.

7. The information processing system according to claim 6, wherein
the holding device includes the second detector that detects biological information of a different type from the biological information detected by the first detector included in the portable information processing terminal.

8. The information processing system according to claim 1 or 2, wherein
the portable information processing terminal includes a plurality of the first detectors including a camera, and
the control unit acquires the biological information in one of a first acquisition mode in which the biological information detected by the plurality of the first detectors including the camera is acquired, or a second acquisition mode in which the biological information detected by the plurality of the first detectors excluding the camera is acquired, depending on the holding device or a position at which the holding device is installed.

9. The information processing system according to claim 8, wherein
one of the first acquisition mode or the second acquisition mode is set in the holding device.

10. The information processing system according to claim 1 or 2, wherein
the control unit starts acquiring the biological information, in accordance with one of illuminance around the holding device or time.

11. A program for causing a computer to perform a process of
acquiring biological information detected by a first detector, when a portable information processing terminal including the first detector that detects the biological information is held by a holding device including a holding unit that holds the portable information processing terminal and a communication unit that communicates with the portable information processing terminal, and communication between the portable information processing terminal and the holding device has been established via the communication unit.

12. An information processing method implemented by a computer to perform a process of
acquiring biological information detected by a first detector, when a portable information processing terminal including the first detector that detects the biological information is held by a holding device including a holding unit that holds the portable information processing terminal and a communication unit that communicates with the portable information processing terminal, and communication between the portable information processing terminal and the holding device has been established via the communication unit.
